(19) Europäisches Patentamt
European Patent Office
Office européen des brevets

(11) Veröffentlichungsnummer: **0 000 057**
**B1**

(12) # EUROPÄISCHE PATENTSCHRIFT

(45) Veröffentlichungstag der Patentschrift: **14.07.82**

(51) Int. Cl.³: **C 07 H 15/22**

(21) Anmeldenummer: **78100099.7**

(22) Anmeldetag: **06.06.78**

(54) **Selektiv geschützte 4,6-Di-O-(aminoglycosyl)-1,3-diaminocyclitole.**

(30) Priorität: **10.06.77 DE 2726197**
**10.06.77 DE 2726208**

(43) Veröffentlichungstag der Anmeldung: **20.12.78 Patentblatt 78/1**

(45) Bekanntmachung des Hinweises auf die Patenterteilung: **14.07.82 Patentblatt 82/28**

(84) Benannte Vertragsstaaten: **CH DE FR GB**

(56) Entgegenhaltungen:
**DE-A-2 512 587**
**FR-A-2 240 015**
**FR-A-2 263 251**
**US-A-3 997 524**

(73) Patentinhaber: **BAYER AG, Zentralbereich Patente, Marken und Lizenzen, D-5090 Leverkusen 1, Bayerwerk (DE)**

(72) Erfinder: **Voss, Eckart, Dr., Morgengraben 10, D-5000 Köln 80 (DE)**
Erfinder: **Stadler, Peter, Dr., Ohligserstrasse 85, D-5657 Haan (DE)**
Erfinder: **Petersen, Uwe, Dr., Auf dem Forst 4, D-5090 Leverkusen 1 (DE)**
Erfinder: **Kabbe, Hans-Joachim, Dr., Walter-Flex-Strasse 16, D-5090 Leverkusen 1 (DE)**
Erfinder: **Metzger, Karl Georg, Dr., Pahlkestrasse 15, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 000 057 B1

BUNDESDRUCKEREI BERLIN

Selektiv geschützte 4,6-Di-O-(aminoglykosyl)-1,3-diaminocyclitole

Die Erfindung betrifft neue, selektiv acylierte oder sulfenylierte 4,6-Di-O-(aminoglykosyl)-1,3-diaminocyclitole, die als Zwischenprodukte zur Herstellung wertvoller neuer und bekannter Antibiotika dienen können.

Bei den neuen Aryl- oder Sulfenylderivaten der 4,6-Di-O-(aminoglykosyl)-1,3-diaminocyclitole handelt es sich um an 4 der 5 Aminogruppen durch Reste $-SR'$ oder $-CO-A$ blockierte Aminoglykoside aus der Gruppe Sisomicin, Verdamicin, Gentamicin $C_{1a}$ und Gentamicin $C_2$, wobei R' Phenyl, durch 1 – 3 Substituenten aus der Reihe Nitro, $C_1-C_4$-Alkyl, $C_1-C_4$-Alkoxy, $C_1-C_4$-Alkylcarbonyl oder Phenyl oder durch 1 – 5 Halogenatome (vorzugsweise Chlor) substituiertes Phenyl, Di- oder Triphenylmethyl und A einen der Reste

$-(CH_2)_n-B$ oder 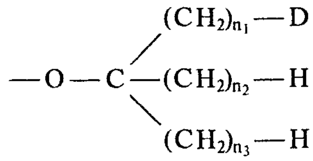

bedeuten, in denen

B und D für Wasserstoff, Phenyl oder durch einen oder zwei Substituenten aus der Reihe Nitro, $C_1-C_4$-Alkyl, $C_1-C_4$-Alkoxy, Phenyl oder Halogen (vorzugsweise Chlor) substituiertes Phenyl und

n, $n_1$, $n_2$ und $n_3$ unabhängig für eine Zahl von 0 – 5 stehen,

mit der Maßgabe, daß die Aminogruppe in 6'-Stellung eine der Schutzgruppen $-SR'$ oder $-CO-A$ aufweist.

Als Beispiele für Reste R' seien der o-Nitrophenylsulfenylrest und der 2,4,5-Trichlorphenylsulfenylrest genannt.

Aus US-PS 3 997 524 sind zwar bereits an vier der fünf Aminogruppen durch Arylreste geschützte Derivate des Sisomicins und Verdamicins bekannt. Die Schutzgruppen aufweisenden Stickstoffatome sind jedoch jene in den 1,3,2'- und 3''-Positionen, während die Aminogruppe in der 6'-Position nicht blockiert ist. Bei den erfindungsgemäßen Verbindungen ist jedoch gerade die 6'-Position zwingend mit einer Schutzgruppe versehen. Gemäß US-PS 3 997 524 werden die tetra-blockierten Aminoglykoside hergestellt, indem man zunächst die reaktivste 6'-Aminogruppe mit einer leicht abspaltbaren Schutzgruppe versieht, danach die restlichen vier Aminogruppen acyliert und die Schutzgruppen in der 6'-Position wieder abspaltet. Es wird somit keinerlei Anregung gegeben, Derivate herzustellen, bei denen eine der Aminogruppen in der 1,3,2'- oder 3''-Position nicht geschützt ist.

In FR-A-2 240 015 und 2 263 251 werden Aminoglykoside beschrieben, welche in der 2'- und/oder 6'-Position Schutzgruppen aufweisen. Es findet sich jedoch keinerlei Hinweis auf Derivate mit vier Schutzgruppen und dem Fachmann wird auch kein Weg gewiesen, auf welchem er zu Aminoglykosid-Derivaten gelangen könnte, bei denen selektiv eine Aminogruppe ungeschützt ist.

Erfindungsgemäß bevorzugt sind Derivate von Sisomicin der Formel (I)

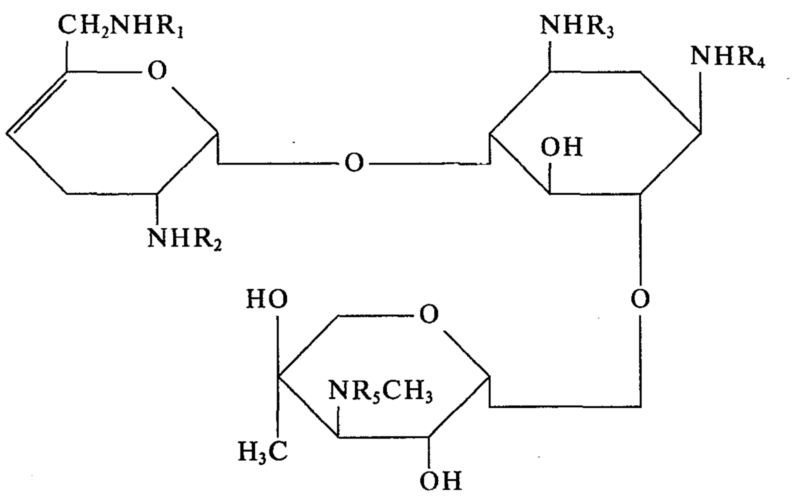

(I)

wobei

| | |
|---|---|
| $R_1$ | für einen Rest —SR′ oder —CO—A steht, und |
| $R_2$, $R_3$, $R_4$ und $R_5$ | Wasserstoff, einen Rest —SR′ oder —CO—A darstellen, mit der Maßgabe, daß einer und nur einer der Reste $R_2$—$R_5$ für Wasserstoff steht, und |
| R′ und A | die oben angegebene Bedeutung haben. |

Beispiele für erfindungsgemäß bevorzugte Verbindungen sind

2′,3,3″,6′-Tetra-N-(o-nitrophenylsulfenyl)-sisomicin,
1,2′,3,6′-Tetra-N-acetyl-sisomicin,
1,3,3″,6′-Tetra-N-(t-butoxycarbonyl)-sisomicin,
1,2′,3,6′-Tetra-N-(ethoxycarbonyl)-sisomicin,
1,2′,3″,6′-Tetra-N-(o-nitrophenylsulfenyl)-sisomicin und
1,3,3″,6′-Tetra-N-(o-nitrophenylsulfenyl)-sisomicin.

Wie sich aus US-PS 3 997 524 ergibt, ist der Erfindungsgedanke aber auch auf Verdamicin übertragbar: Der Literaturstelle ist zu entnehmen, daß auch beim Verdamicin die Aminogruppe in der 6′-Position die reaktivste ist. Ähnliches gilt für Gentamicin $C_{1a}$ und $C_2$, wie die Beispiele 9 und 10 der europäischen Patentanmeldung 80 108 216.5 zeigen.

Die erfindungsgemäßen Derivate werden erhalten, indem man Sisomicin, Verdamicin, Gentamicin $C_{1a}$, Gentamicin $C_2$ oder deren Säureadditionssalze, mit etwa 4 Äquivalenten einer Verbindung der Formel

R′—S—G

in der

R′ die oben angegebene Bedeutung besitzt und
G Halogen oder eine bei Sulfenylierungsreaktionen gebräuchliche Abgangsgruppe, vorzugsweise einen aktivierenden Esterrest, bezeichnet,

oder mit etwa 4 Äquivalenten einer Verbindung der Formel

A—CO—G′

in der

A die oben genannte Bedeutung besitzt und
G′ Halogen oder eine bei Acylierungsreaktionen gebräuchliche Abgangsgruppe, vorzugsweise einen aktivierenden Esterrest, oder eine Gruppe —O—CO—A mit der obigen Bedeutung für A bezeichnet,

in einem inerten Lösungsmittel, gegebenenfalls unter Zusatz von Wasser, bei Temperaturen zwischen etwa −30°C und +50°C, vorzugsweise zwischen etwa 0°C und +25°C, in Gegenwart einer Base umsetzt und das Reaktionsprodukt in üblicher Weise aufarbeitet.

Verwendet man Sisomicin und o-Nitrophenylsulfensäure-p-nitrophenylester als Ausgangsstoffe, so kann der Reaktionsablauf durch das folgende Schema wiedergegeben werden:

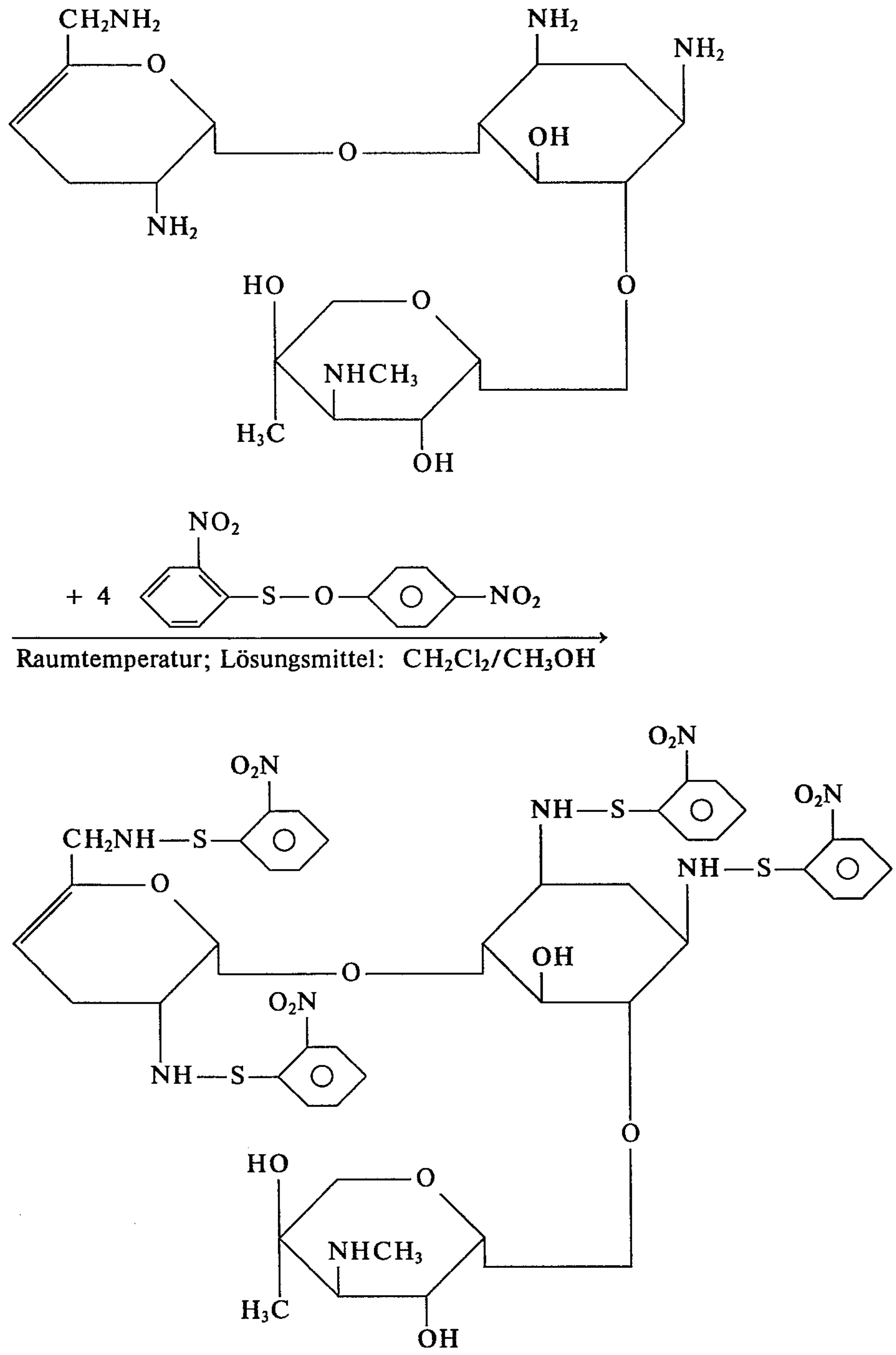


Für die Darstellung der erfindungsgemäßen Verbindungen auf dem oben geschilderten Wege durch Umsetzung der ungeschützten 4,6-Di-O-(aminoglykosyl)-1,3-diaminocyclitole mit einem Sulfenylierungsreagenz finden vorzugsweise Sulfenylierungsreagenzien Verwendung, in denen G Chlor oder den p-Nitrophenyloxyrest bezeichnet. Als Beispiele seien genannt:

Tritylsulfenylchlorid, o-Nitrophenylsulfenylchlorid,
2,4-Dinitrophenylsulfenylchlorid, 2,4,5-Trichlorphenylsulfenylchlorid,
Pentachlorphenylsulfenylchlorid und o-Nitrophenylsulfensäure-p-nitrophenylester,
2,4-Dinitrophenylsulfenyl-p-nitrophenylester,
2,4,5-Trichlorphenylsulfensäure-p-nitrophenylester und
Pentachlorphenylsulfensäure-p-nitrophenylester.

Diese reaktiven Sulfensäurederivate sind entweder bereits bekannt (siehe z. B. Houben—Weyl, Methoden der Organischen Chemie, Band XV, 1, Seiten 203—222, Georg Thieme Verlag, Stuttgart,

1974) oder können nach analogen Verfahren wie die bereits bekannten Verbindungen hergestellt werden.

Bevorzugte Acylierungsreagenzien sind solche, bei denen G' Chlor oder den Rest —O—CO—A, mit der oben angegebenen Bedeutung für A, bezeichnet. Beispielhaft seien genannt: Acetanhydrid, Acetylchlorid und Di-t-Butylpyrocarbonat, wobei zur Darstellung von N-Alkyl-oxycarbonyl-derivaten des Sisomicins die Verwendung von Dialkylpyrocarbonaten als Blockierungsreagenz im allgemeinen besonders zu bevorzugen ist.

Als Verdünnungsmittel für die Umsetzung mit Sulfensäurehalogeniden kommen sowohl inerte organische Lösungsmittel wie Chloroform und Toluol, vorzugsweise aber mit Wasser mischbare Lösungsmittel wie Dioxan, DMF und Dimethoxyethan sowie deren Gemische mit Wasser in Frage.

Die Umsetzungen mit aktivierten Estern der obengenannten Sulfensäuren erfolgen vorzugsweise in inerten organischen Lösungsmitteln wie $CHCl_3$, DMF, Pyridin oder Gemischen von solchen Lösungsmitteln mit Alkoholen, vorzugsweise Methanol oder Ethanol. Die Darstellung der erfindungsgemäßen Acylverbindungen erfolgt in beliebigen inerten organischen Lösungsmitteln oder in Gemischen organischer Lösungsmittel mit Wasser, wobei Gemische von Methanol, Ethanol oder Aceton mit Wasser zu bevorzugen sind.

Als Basen können alle in der organischen Chemie üblichen basischen Verbindungen wie z. B. Triethylamin, Pyridin, Diazabicyclononen eingesetzt werden; vorzugsweise werden aber Alkali-hydroxide bzw. -carbonate wie z. B. Natronlauge oder Natriumcarbonat verwendet.

Die Sulfenylierungen bzw. Acylierungen werden bei Temperaturen zwischen etwa —30° C und +50° C, vorzugsweise zwischen etwa 0° C und +25° C, durchgeführt.

Die Umsetzungen können sowohl bei Normaldruck, aber auch bei erhöhtem Druck durchgeführt werden. Im allgemeinen arbeitet man bei Normaldruck.

Bei der Sulfenylierung bzw. Acylierung der Aminoglycoside können auch die Säureadditionssalze aus den 4,6-Di-O-(aminoglykosyl)-1,3-Diaminocyclitolen mit anorganischen und organischen Säuren, vorzugsweise die Chloride und Sulfate, eingesetzt werden.

In diesem Falle muß die Menge der verwendeten Hilfsbase entsprechend der Zahl der in Salzform vorliegenden Aminogruppen variiert werden.

Die erfindungsgemäßen Derivate sind wertvolle Zwischenprodukte bei der Darstellung von Derivaten der 4,6-Di-O-(aminoglykosyl)-1,3-diaminocyclitole Gentamicin $C_{1a}$, Gentamicin $C_2$, Sisomicin und Verdamicin.

Diese Antibiotika sind wertvolle Substanzen zur wirkungsvollen Bekämpfung bakterieller Infektionen. Ihre hohe Wirksamkeit ist jedoch häufig verbunden mit relativ großer Nephro- und Ototoxizität; hinzu kommt Resistenzbildung der bekämpften Keime. Aus diesen Gründen ist es wünschenswert, Derivate der Aminoglykosid-Antibiotika mit verbesserten Eigenschaften herzustellen, die bei verringerter Toxizität eventuell auch die Bekämpfung resistenter Keime ermöglichen. Als Substanzen mit derartig verbesserten Eigenschaften sind Verbindungen wie 1-N-Acetylsisomicin und 1-N-Ethylsisomicin bekanntgeworden (DE-OS 2 437 160).

Die Darstellung mono-N-substituierter Sisomicin-Derivate erweist sich jedoch ausgehend von ungeschütztem Sisomicin als schwierig, da im Sisomicin-Molekül fünf Aminogruppen vergleichbarer Reaktivität vorhanden sind. Man gelangt daher immer zu Reaktionsgemischen, was aufwendige chromatographische Verfahren zur Isolierung der gewünschten Verbindung notwendig macht.

Erfindungsgemäß werden nunmehr Derivate von Aminoglysid-Antibiotika mit einer selektiv ungeschützten Aminogruppe zur Verfügung gestellt, wobei die Natur der Schutzgruppen so beschaffen ist, daß sowohl Alkylierungen als auch Acylierungen (in glatter Reaktion und mit hohen Ausbeuten) an den freien Stickstoffatomen möglich sind und eine anschließende einfache und schonende Entblockierung durchgeführt werden kann, ohne daß aufwendige chromatographische Reinigungsoperationen erforderlich werden.

Die Sulfenyl-Schutzgruppen können sowohl mit Nucleophilen wie z. B. $H_2S$, Thiophenol usw. als auch durch schwache Säuren abgespalten werden, d. h. unter Bedingungen, unter denen die neu eingeführten Acyl- oder Alkylreste stabil sind. Werden Acylreste, wie z. B. Acetyl-, Ethyloxycarbonyl- oder t-Butyloxycarbonylgruppen als Schutzgruppen verwendet, kann die Deblockierung mit wäßrigem Alkali- oder Erdalkalihydroxid oder mit Säuren wie Trifluoressigsäure, Perchlorsäure oder Bortrifluoridetherat in organischen Lösungsmitteln bzw. Mischungen von organischen Lösungsmitteln mit Wasser erfolgen. Auch hier werden bei entsprechender Wahl von Acyl-Schutzgruppen und Abspaltungsreagenzien neu eingeführte Alkyl- bzw. Acylreste bei der Deblockierung nicht angegriffen.

Die selektive Abspaltung der Schutzgruppen kann sowohl mit äquivalenten Mengen Abspaltungsreagens als auch mit einem Überschuß erfolgen, vorzugsweise aber mit einem 20—50fachen Überschuß. Dabei wird die optimale Reaktionszeit dünnschichtchromatographisch ermittelt.

Zur Herstellung von 1-N-Acetyl-sisomicin unter Verwendung eines der erfindungsgemäßen Zwischenprodukte geht man z. B. so vor, daß man ein geschütztes Sisomicinderivat der Formel (I), in der $R_1$, $R_2$, $R_3$ und $R_5$ den o-Nitrophenylsulfenylrest bezeichnen, mit 1 Äquivalent Essigsäure-p-nitrophenylester in Pyridin umsetzt, die o-Nitrophenylsulfenylschutzgruppen mit überschüssigem Schwefelwasserstoff in Alkohol bei pH 1—3 abspaltet und 1-N-Acetylsisomicin durch Behandlung mit

einem basischen Ionenaustauscher isoliert.

Zur Darstellung von 2'-N-Acetylsisomicin setzt man z. B. 1 Mol einer Verbindung der Formel (I), in der $R_1$, $R_3$, $R_4$ und $R_5$ tert.-Butyloxycarbonyl-Reste bezeichnen, mit 1 Äquivalent Essigsäureanhydrid in Methanol um und spaltet nach Beendigung der Acylierungsreaktion die Schutzgruppen mit überschüssigem Bortrifluorid-Etherat in Aceton ab und isoliert das entstandene 2'-N-Acetylsisomicin durch Behandlung mit einem basischen Ionenaustauscher.

Besonders vorteilhaft gelingt die Herstellung von 1-N-Formylsisomicin. Dazu setzt man 2',3,3'',6'-Tetra-N-(o-nitro-phenylsulfenyl)-sisomicin, das durch Sulfenylierung von Sisomicin bzw. dessen Säureadditionssalzen zugänglich ist, mit etwa 1 bis 5 Mol Formylierungsmittel um, spaltet die o-Nitrophenylsulfenylgruppen mit schwefelhaltigen, nucleophilen Reagentien wie z. B. $H_2S$ oder Thiophenol ab, arbeitet den Reaktionssatz in üblicher Weise auf das freie 1-N-Formylderivat hin auf und überführt dieses gegebenenfalls in die pharmazeutisch verwendbaren Salze. Die Formylierung des Sulfenylderivates erfolgt vorzugsweise in Pyridin als Verdünnungsmittel unter Verwendung von Ameisensäure-p-nitrophenylester als Formylierungsmittel.

Anstelle der o-Nitrophenylsulfenyl-Derivate können mit gutem Erfolg auch andere Sulfenylderivate, die in analoger Weise durch Umsetzung von Sisomicin mit entsprechenden Sulfonsäurechloriden bzw. den Sulfonsäuren-p-nitrophenylestern erhältlich sind, eingesetzt werden. Als Beispiele seien genannt: Tritylsulfenyl-, Pentachlorphenylsulfenyl- und 2,4-Dinitrophenylsulfenylsisomicin-Derivate.

Neben Ameisensäure-p-nitrophenylester eignet sich auch Ameisensäure-essigsäure-anhydrid besonders gut zur Formylierung des Sulfenylderivates.

## Beispiel 1

### 1,3,3'',6'-Tetra-N-(t-butoxycarbonyl)-sisomicin

450 mg Sisomicin werden in 10 ml Wasser gelöst. Nach Zugabe von 10 ml Methanol versetzt man unter gutem Rühren mit 870 mg Pyrokohlensäure-di-t-butylester. Nach 1,3 Stunden Rühren bei Raumtemperatur versetzt man mit 5 ml Wasser, filtriert und dampft das Filtrat im Vakuum zur Trockene ein. Man löst den Rückstand in Methanol und fällt das gewünschte Produkt durch Zugabe von Ether und Petrolether. Ausbeute = 600 mg.

$^{13}$C-NMR ($CD_2OD$):
$\delta$ = 66,01 (C-3''); 52,23 (C-1); 51,67 (C-3); 48,23 (C-2'); 43,74 (C-6'); 157,69 ($\geq$ c = 0) ppm.

## Beispiel 2

### 1,2',3,6'-Tetra-N-(ethoxycarbonyl)-sisomicin

1,1 g Sisomicin werden in 50 ml Ethanol und 70 ml Wasser gelöst. Nach Abkühlen auf −10° C tropft man unter gutem Rühren 1,35 ml Pyrokohlensäurediethylester dazu. Nach weiteren 2$^1/_2$ Stunden bei −10° C wird mit 100 ml Wasser versetzt. Man extrahiert anschließend mit 150 ml Petrolether und dampft die wäßrige Phase im Vakuum zur Trockene ein. Der Rückstand wird in Methanol gelöst. Durch Zugabe von überschüssigem Ether und Petrolether wird das gewünschte Produkt ausgefällt. Ausbeute = 1,5 g (91%).

$^{13}$C-NMR ($CD_3OD/CDCl_3$):
$\delta$ = 50,86 (C-1); 49,90 (C-2); 46,33 (C-2'); 42,87 (C-6'); 157,94, 157,73, 157,29 und 157,22 ($\geq$ c = 0) ppm.

## Beispiel 3

### 1,2',3,6'-Tetra-N-acetyl-sisomicin

1,1 g Sisomicin werden in 120 ml Wasser gelöst. Nach Zusatz von 60 ml Methanol tropft man unter Rühren 2,5 ml Acetanhydrid dazu. Nach 15 Minuten wird im Vakuum zur Trockene eingedampft. Den Rückstand löst man in 10 ml Methanol und tropft diese Lösung in eine Mischung von 30 ml Ether und 30 ml Petrolether, wobei das gewünschte Produkt ausfällt. Ausbeute = 1,43 g, MS: m/e = 615.

$^{13}$C-NMR ($CD_3OD$):
$\delta$ = 50,14 (C-1); 49,20 (C-3); 46,88 (C-2'); 42,26 (C-6'); 173,24, 173,13, 172,63 ($\geq$ c = 0) ppm.

## Beispiel 4
## (Verwendungsbeispiel)

### 3''-N-p-Aminobenzylsisomicin

1,3 g des Produktes von Beispiel 3 in 20 ml Dimethylformamid werden mit 2,5 g Silberoxid und 2,2 g p-Nitrobenzylbromid für 1 Stunde bei Raumtemperatur gerührt. Anschließend verdünnt man mit 50 ml Chloroform, rührt kurz mit wenig Holzkohle und filtriert. Das Filtrat wird im Vakuum zur Trockene eingedampft und der Rückstand aus Chloroform mit Petrolether umgefällt. Ausbeute = 1,1 g.

Zur Reduktion der Nitrogruppe löst man 200 mg des oben erhaltenen Produktes in 2 ml Wasser und gibt nach Zusatz von 0,06 ml Essigsäure 400 mg Eisenpulver dazu. Man erhitzt für 10 Minuten auf 70°C und läßt anschließend noch 1 Stunde bei Raumtemperatur reagieren. Zur Aufarbeitung wird filtriert, das Filtrat eingedampft und der Rückstand in 5 ml Methanol und 5 ml Wasser mit Holzkohle entfärbt. Nach Filtrieren der Lösung wird im Vakuum eingedampft. Zur Abspaltung der N-Acetylgruppen erhitzt man den so erhaltenen Rückstand in 2 ml Wasser mit 1 g Bariumhydroxidoctahydrat für 8 Stunden am Rückfluß. Man läßt erkalten, fällt die Bariumsalze mit Kohlendioxid und filtriert. Eindampfen des Filtrats und Fällen des erhaltenen Rückstands aus Methanol mit Ether liefert die Titelverbindung. Ausbeute = 95 mg.

## Beispiel 5
## (Verwendungsbeispiel)

### a) 2'-N-Acetyl-1,3,3'',6'-tetra-N-t-butoxycarbonylsisomicin

175 mg 1,3,3'',6'-Tetra-N-t-butoxycarbonyl-sisomicin werden in 7 ml Methanol gelöst. Diese Lösung versetzt man nacheinander mit 7 ml Aceton sowie 2 ml Acetanhydrid. Nach 2 Stunden wird im Vakuum vom Lösungsmittel befreit und der dabei erhaltene Rückstand in Methanol gelöst. Man fällt das gewünschte Produkt durch Zugabe von überschüssigem Ether und Petrolether. Ausbeute = 170 mg.

$^{13}C$-NMR ($CD_3OD/CDCl_3$):
$\delta$=56,72 (C-2'); 23,14 und 175,80 (Acetyl) ppm.

### b) 2'-N-Acetylsisomicin

Zur Abspaltung der t-Butoxycarbonylschutzgruppen löst man 150 mg des nach a) erhaltenen Produktes in 3 ml Aceton und versetzt mit 0,6 ml Bortrifluorid-Diethylether-Komplex. Nach 1 Stunde gießt man in eine Mischung von 15 ml Ether und 5 ml Petrolether, wobei das gewünschte Produkt ausfällt. Man entionisiert durch Rühren mit einem basischen Ionenaustauscher ($OH^{\ominus}$-Form) in wäßriger Lösung. Das Harz wird abfiltriert und das Filtrat im Vakuum vom Lösungsmittel befreit. Der Rückstand wird in wenig Methanol gelöst und durch Eingießen in überschüssigen Ether gefällt. Ausbeute = 60 mg.

$^{13}C$-NMR ($D_2O$/Dioxan):
$\delta$=56,67 (C-2'); 21,62 und 177,01 (Acetyl) ppm.

## Beispiel 6
## (Verwendungsbeispiel)

### 2'-N-Acetylsisomicin

265 mg (0,25 mMol) 1,3,3'',6'-Tetra-N-NPS-sisomicin (Beispiel 9) und 47 mg Essigsäure-p-nitrophenylester werden in 1,25 ml absol. Pyridin 1 Stunde bei Raumtemperatur gerührt, das Lösungsmittel abgedampft, der Rückstand in 5 ml Dichlormethan aufgenommen und 5 ml einer methanolischen Schwefelwasserstofflösung (bei 0°C gesätt.) sowie 0,7 ml einer gesätt. (20°C) Lösung von HCl in Methanol 1 Minute bei Raumtemperatur geschüttelt, das Reaktionsgemisch eingedampft und der Rückstand mit 20 ml Wasser digeriert. Das Digerat wird filtriert, das Filtrat zweimal mit Ether ausgeschüttelt, die wäßrige Phase über 10 ml basischem Ionenaustauscher ($OH^{\ominus}$-Form) filtriert und eingedampft. Der farblose Eindampfrückstand besteht aus dem reinen 2'-Acetylsisomicin.

Beispiel 7

1,2',3,6'-Tetra-N-(o-nitrophenylsulfenyl)-sisomicin

4,5 g Sisomicin in 10 ml Methanol und 90 ml Dichlormethan werden mit 13 g o-Nitrophenylsulfensäure-p-nitrophenylester versetzt, das Reaktionsgemisch nach 1 Stunde auf 300 ml Methanol gegossen, der Niederschlag mit 50 ml Dichlormethan digeriert und das unlösliche Produkt (3,6 g) getrocknet. Filtrat und Digerat werden vereinigt und an einer 6 x 15 cm $SiO_2$-Säule chromatographiert (Laufmittel: Dichlormethan mit steigendem Methanol-Zusatz, zuletzt 10% Methanol).

Nach Abdampfen des Lösungsmittels werden aus dem Eluat 3,4 g 1,2',3,6'-Tetra-NPS-sisomicin (Gesamtausbeute 7,0 g = 66%) und 2,5 g (21%) Penta-NPS-sisomicin isoliert.

13-C-NMR (δ-6-DMSO):
63,87 (C-3''), 146,07, 147,28, 145,67, 145,11 (jeweils C-1 der arom. Ringe) ppm.

Beispiel 8

a) Penta-N-(o-nitrophenylsulfenyl)-sisomicin

13,84 g (20 mMol) Sisomicinsulfat in 100 ml 1 n-NaOH und 450 ml frisch destilliertem Dioxan werden mit 38 g (0,20 Mol) o-Nitrophenylsulfenylchlorid in 200 ml Dioxan und mit 260 ml 1 n-NaOH versetzt, so daß der pH zwischen 12 und 14 liegt. Der Niederschlag wird abfiltriert, in $CH_2Cl_2/H_2O$ gelöst, und die $CH_2Cl_2$-Phase mit $Na_2SO_4$ getrocknet.

Das Filtrat wird mit $CH_2Cl_2$ versetzt, die wäßrige Phase verworfen und die organische Phase über $Na_2SO_4$ getrocknet. Die vereinigten organischen Phasen werden zur Trockne eingedampft und über 250 g Kieselgel (Säulendurchmesser 8 cm) zuerst mit $CH_2Cl_2$, dann mit $CH_2Cl_2/MeOH$ = 97,5/2,5 filtriert. Das Eluat liefert nach dem Abdampfen des Lösungsmittels 22 g (91%) Penta-N-(o-nitrophenylsulfenyl)-sisomicin als orangefarbenen Schaum.

13-C-NMR ($CDCl_3$):
δ = 124 – 148 (arom. H); 102,30 (C-1''); 99,00 (C-1'); 97,92 (C-4'); 89,05 (C-6); 82,33 (C-4); 57,31 (C-1); 56,73 (C-3) ppm.

b) 3''-N-(o-Nitrophenylsulfenyl)-sisomicin

16,0 g (13,2 mMol) Penta-N-NPS-sisomicin in 80 ml absol. Pyridin werden mit 160 ml Thiophenol versetzt, nach 1 Stunde auf 500 ml Diethylether gegossen, der Niederschlag in Dichlormethan/Methanol = 8/2 aufgenommen und über Kieselgel filtriert (Säule 5,5 x 12 cm, Laufmittel Dichlormethan/Methanol = 8/2, steigender Zusatz des Laufmittelgemisches Methanol/Dichlormethan/20proz. Ammoniak = 4/2/1); die rote Zone liefert nach dem Abdampfen des Lösungsmittels 6,6 g (83%) 3''-N-o-Nitrophenylsulfenyl-sisomicin als tiefroten Schaum.

13-C-NMR ($CD_3OD$):
33,59 ($CH_3N$); 52,23 (C-1); 51,16 (C-3); 53 (C-2'); 43,84 (C-6') ppm.

c) 2',3,3'',6'-Tetra-N-(o-nitrophenylsulfenyl)-sisomicin und
1,2',3'',6'-tetra-N-(o-nitrophenylsulfenyl)-sisomicin

3,0 g (5,0 Mol) 3''-N-NPS-sisomicin in 5 ml Methanol und 45 Mol Dichlormethan werden mit 4,4 g (15,0 Mol) o-Nitrophenylsulfensäure-p-nitrophenylester in 85 ml Dichlormethan versetzt, das Reaktionsgemisch sofort zur Trockne eingedampft, in Dichlormethan aufgenommen und an Kieselgel (Säule 5,5 x 30 cm) mit 200 ml Dichlormethan, dann mit Dichlormethan/Methanol = chromatographiert. Es werden 500 Fraktionen aufgefangen, wobei aus den vereinigten Fraktionen 150 bis 250 das 1,2',3'',6'-Tetra-NPS-sisomicin und aus den Fraktionen 270 bis 500 das 2',3,3'',6'-Tetra-NPS-Derivat jeweils als orangefarbener Schaum erhalten wird.

1,2',3'',6'-Tetra-NPS-sisomicin:
$R_F$ ($CH_2Cl_2/CH_3OH$ = 9/1): 0,62
IR (KBr): 1501, 1360, 1300 (stark); 1587, 1562, 755 (mittel); 1442, 780, 890 (schwach)

2',3,3'',6'-Tetra-NPS-sisomicin:
$R_F$ ($CH_2Cl_2/CH_3OH$ = 9/1): 0,42
IR (KBr): 1500, 1358, 1296 (stark); 1586, 1560, 753 (mittel); 1442, 890, 779 (schwach)

## Beispiel 9

### 1,3,3″,6′-Tetra-N-(o-nitrophenylsulfenyl)-sisomicin

9 g Sisomicin in 20 ml Methanol/180 ml Dichlormethan werden mit 45 g o-Nitrophenylsulfensäure-p-nitrophenylester in 500 ml Dichlormethan versetzt, 10 Stunden bei Raumtemperatur gerührt, auf ein Volumen von 100 ml eingedampft und mit Cyclohexan im Überschuß versetzt. Der Niederschlag (37 g) wird abfiltriert, in 150 ml absol. Pyridin gelöst, das Gemisch mit 200 ml Thiophenol versetzt und nach 1 Stunde auf 500 ml Diethylether gegossen. Der Niederschlag wird in Dichlormethan/Methanol = 8/2 aufgenommen und über Kieselgel filtriert (Säule 5,5 x 12 cm, Laufmittel Dichlormethan/Methanol = 8/2, steigender Zusatz des Laufmittelgemisches Methanol/Dichlormethan/20proz. Ammoniak = 4/2/1). Die rote Zone wird eingedampft; 1800 mg des entstandenen Schaums werden in 3 ml Methanol und 27 ml Dichlormethan gelöst, mit 2,7 g o-Nitrophenylsulfensäure-p-nitrophenylester und 100 ml Methanol versetzt, eingedampft und der Rückstand an 100 g Kieselgel mit Dichlormethan/Methanol = 98/2 chromatographiert. Die gelbe Zone liefert nach dem Eindampfen 1,5 g 1,3,3″,6′-Tetra-NPS-sisomicin.

$R_F$ ($CH_2Cl_2/CH_3OH$ = 9/1): 0,37
13-C-NMR(d-6-Dioxan): 124—147 (arom. C); 146,8 (C-5′); 103,85 (C-1″); 102,40 (C-1′); 88,89 (C-6); 76,48 (C-5) ppm.

## Beispiel 10
(Verwendungsbeispiel)

### 1-N-Methoxyacetyl-sisomicin

265 g (0,25 mMol) 2′,3,3″,6′-Tetra-NPS-sisomicin (Beispiel 8c) und 58 mg Methoxyessigsäure-p-nitrophenylester werden in 1,25 ml absol. Pyridin 1 Stunde bei Raumtemperatur gerührt, das Lösungsmittel abgedampft, der Rückstand in 5 ml Dichlormethan aufgenommen und mit 5 ml einer methanolischen Schwefelwasserstofflösung (bei 0° C gesätt.) sowie 0,7 ml einer gesätt. (20° C) Lösung von HCl in Methanol 1 Minute bei Raumtemperatur geschüttelt, das Reaktionsgemisch mit einigen Tropfen wäßriger Ammoniaklösung neutralisiert, eingedampft und der Rückstand mit 20 ml Wasser digeriert. Das Digerat wird filtriert, das Filtrat zweimal mit Ether ausgeschüttelt, die wäßrige Phase über 10 ml basischem Ionenaustauscher ($OH^{\ominus}$-Form) filtriert und eingedampft. Der farblose Eindampfrückstand besteht aus dem reinen 1-N-Methoxyacetyl-sisomicin.

$R_F$ ($CHCl_3/CH_3OH$/20% $NH_4OH$ = 2/4/1): 0,53
IR (KBr): 1650, 1105, 1048, 1000 (stark)

## Beispiel 11
(Verwendungsbeispiel)

### 1-N-Acetyl-sisomicin

265 g (0,25 mMol) 2′,3,3″,6′-Tetra-NPS-sisomicin (Beispiel 8c) werden mit 47 mg Essigsäure-p-nitrophenylester wie für das 1-N-Methoxyacetylsisomicin beschrieben, umgesetzt. Nach Aufarbeitung des Reaktionsansatzes wird ganz analog das reine 1-N-Acetylsisomicin durch Eindampfen des wäßrigen Ionenaustauscher-Eluats erhalten.

13-C-NMR ($CD_3OD$):
23,03 ($CH_3CO$); 173,94 (C=0); 51,14 (C-3); 48,41 (C-2′); 150,62 (C-5); 44,21 (C-6′); 65,89 (C-3″).

## Beispiel 12
(Verwendungsbeispiel)

### 3-N-Acetylsisomicin

265 mg (0,25 mMol) 1,3,3″,6′-Tetra-NPS-sisomicin (Beispiel 9) werden mit 47 mg Essigsäure-p-nitrophenylester wie für das 1-N-Acetyl-sisomicin beschrieben umgesetzt. Nach identischer Aufarbeitung des Ansatzes wird das reine 3-N-Acetyl-sisomicin als einziges Produkt isoliert.

13-C-NMR ($CD_3OD$):

23,22 ($CH_3CO$); 173,13 (CO); 35,33 (C-2); 82,23 (C-4); 48,50 (C-2'); 148,42 (C-5'); 43,73 (C-6'); 66,01 (C-3'').

### Beispiel 13
### (Verwendungsbeispiel)

### 3''-N-Acetylsisomicin

265 mg (0,25 mMol) 1,2',3,6'-Tetra-NPS-sisomicin (Beispiel 8c) werden mit 47 mg Essigsäure-paranitrophenylester wie für das 1-N-Acetyl-sisomicin beschrieben umgesetzt. Nach identischer Aufarbeitung des Ansatzes wird das reine 3''-N-Acetylsisomicin als einziges Produkt isoliert.

13-C-NMR-($D_2O$/d-6-Dioxan):
Rotamerengemisch:
22,37/22,18 ($CH_3CO$); 177,13/176,76 (CO); 65,67/62,06 (C-3''); 21,59/21,36 (C(OH)$-CH_3$); 32,97/30,35 ($CH_3N$).

### Beispiel 14
### (Verwendungsbeispiel)

### 1-N-Formylsisomicin

265 mg des nach Beispiel 8c) erhaltenen 2',3,3'',6'-Tetra-N-(o-nitro-phenylsulfenyl)-sisomicins und 44 g Ameisensäure-p-nitrophenylester werden in 1 ml Pyridin gelöst. Man läßt 1 Stunde bei Raumtemperatur stehen, dampft den Ansatz im Vakuum ein und nimmt in 2 ml Dichlormethan auf. Die Lösung wird mit 7,5 ml einer methanolischen, bei 0°C gesättigten $H_2S$-Lösung sowie 0,75 ml einer bei 20°C gesättigten, methanolischen Chlorwasserstofflösung versetzt und nach 1 Minute mit konz. Ammoniaklösung neutralisiert. Man dampft den Ansatz im Vakuum ein, digeriert den Rückstand mit 10 ml Wasser und filtriert. Das Filtrat wird zweimal mit Ether gewaschen, die wäßrige Phase über 12 ml basischen Ionenaustauscher in der $OH^{\ominus}$-Form filtriert und eingedampft.
Ausbeute: 102 mg 1-N-Formylsisomicin

IR (KBr-Preßling): 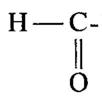-Bande bei 1663 $cm^{-1}$.

**Patentansprüche**

1. An 4 der 5 Aminogruppen durch Reste $-SR'$ oder $-CO-A$ blockierte Aminoglykoside aus der Gruppe Sisomicin, Verdamicin, Gentamicin $C_{1a}$ und Gentamicin $C_2$, wobei R' Phenyl, durch 1–3 Substituenten aus der Reihe Nitro, $C_1-C_4$-Alkyl, $C_1-C_4$-Alkoxy, $C_1-C_4$-Alkylcarbonyl oder Phenyl oder durch 1–5 Halogenatome substituiertes Phenyl, Di- oder Triphenylmethyl und A einen der Reste

$-(CH_2)_n-B$ oder 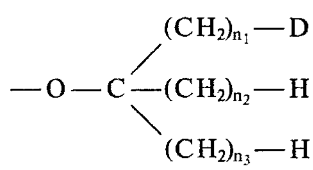

bedeuten, in denen

B und D für Wasserstoff, Phenyl oder durch einen oder zwei Substituenten aus der Reihe Nitro, $C_1-C_4$-Alkyl, $C_1-C_4$-Alkoxy, Phenyl oder Halogen substituiertes Phenyl und

n, $n_1$, $n_2$ und $n_3$ unabhängig für eine Zahl von 0–5 stehen,

dadurch gekennzeichnet, daß die Aminogruppe in 6'-Stellung eine der Schutzgruppen $-SR'$ oder $-CO-A$ aufweist.

2. Verbindung gemäß Anspruch 1 der Formel

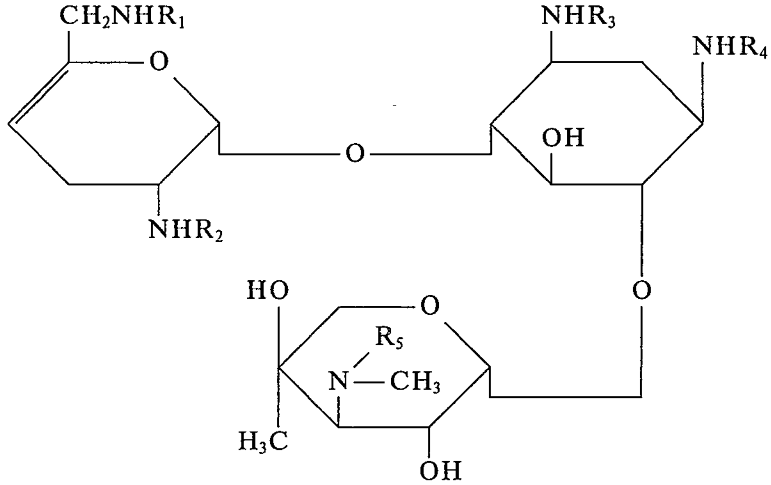

wobei

| | |
|---|---|
| $R_1$ | für einen Rest $-SR'$ oder $-CO-A$ steht und |
| $R_2$, $R_3$, $R_4$ und $R_5$ | Wasserstoff, einen Rest $-SR'$ oder $-CO-A$ darstellen, mit der Maßgabe, daß einer und nur einer der Reste $R_2-R_5$ für Wasserstoff steht, und |
| R' und A | die in Anspruch 1 angegebene Bedeutung haben. |

3. 2',3,3'',6'-Tetra-N-(o-nitrophenylsulfenyl)-sisomicin.
4. 1,2',3,6'-Tetra-N-acetyl-sisomicin.
5. 1,3,3'',6'-Tetra-N-(t-butoxycarbonyl)-sisomicin.
6. 1,2',3,6'-Tetra-N-(äthoxycarbonyl)-sisomicin.
7. 1,2',3'',6'-Tetra-N-(o-nitrophenylsulfenyl)-sisomicin.
8. 1,3,3'',6'-Tetra-N-(o-nitrophenylsulfenyl)-sisomicin.

**Claims**

1. Aminoglycosides, which are blocked on 4 of the 5 amino groups by the radicals $-SR'$ or $-CO-A$, from the group comprising sisomycin, verdamycin, gentamycin $C_{1a}$ and gentamycin $C_2$, wherein R' denotes phenyl, phenyl which is substituted by 1–3 substituents from the series comprising nitro, $C_1-C_4$-alkyl, $C_1-C_4$-alkoxy, $C_1-C_4$-alkylcarbonyl or phenyl or by 1–5 halogen atoms, diphenylmethyl or triphenylmethyl and A denotes one of the radicals

$-(CH_2)_n-B$ or 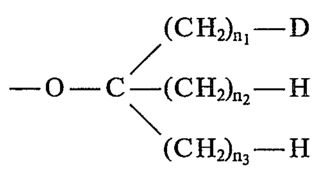

in which

| | |
|---|---|
| B and D | represent hydrogen, phenyl or phenyl which which is substituted by one or two substituents from the series comprising nitro, $C_1-C_4$-alkyl, $C_1-C_4$-alkoxy, phenyl or halogen, and |
| n, $n_1$, $n_2$ and $n_3$ | independently represent a number from 0–5, |

characterised in that the amino group in the 6'-position exhibits one of the protective groups $-SR'$ or $-CO-A$.

2. Compound according to Claim 1 of the formula

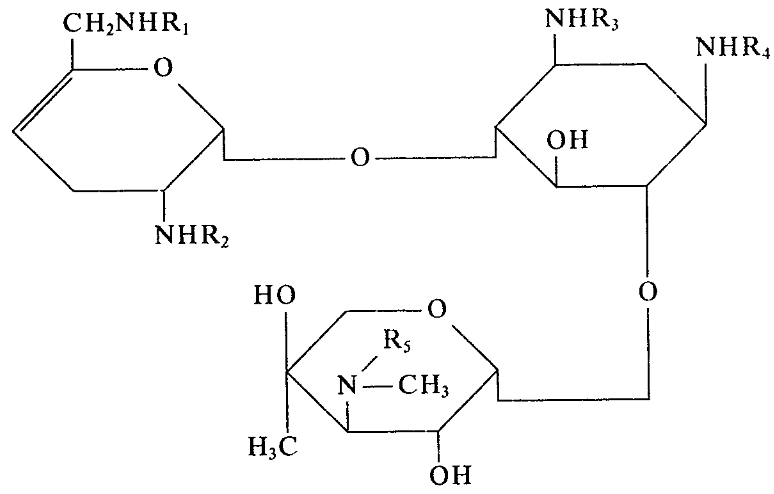

wherein

| | |
|---|---|
| $R_1$ | represents a radical $-SR'$ or $-CO-A$ and |
| $R_2$, $R_3$, $R_4$ and $R_5$ | represent hydrogen, a radical $-SR'$ or $-CO-A$, with the proviso that one and only one of the radicals $R_2-R_5$ represents hydrogen, and |
| R' and A | have the meaning indicated in Claim 1. |

3. 2',3,3'',6'-Tetra-N-(o-nitrophenylsulphenyl)-sisomycin.
4. 1,2',3,6'-Tetra-N-acetyl-sisomycin.
5. 1,3,3'',6'-Tetra-N-(t-butoxycarbonyl)-sisomycin.
6. 1,2',3,6'-Tetra-N-(ethoxycarbonyl)-sisomycin.
7. 1,2',3'',6'-Tetra-N-(o-nitrophenylsulphenyl)-sisomycin.
8. 1,3,3'',6'-Tetra-N-(o-nitrophenylsulphenyl)-sisomycin.

**Revendications**

1. Aminoglycosides du groupe de la sisomicine, de la verdamicine, de la gentamicine $C_{1a}$ et de la gentamicine $C_2$, protégés sur quatre des cinq groupes amino par des restes $-SR'$ ou $-CO-A$, ou R' est un groupe phényle, phényle substitué par 1 à 3 substituants de la série nitro, alkyle en $C_1$ à $C_4$, alkoxy en $C_1$ à $C_4$, (alkyle en $C_1$ à $C_4$)-carbonyle ou phényle, ou par 1 à 5 atomes d'halogènes, di- ou triphénylméthyle et A désigne l'un des restes:

$$-(CH_2)_n-B \quad \text{ou} \quad -O-C\begin{matrix}(CH_2)_{n_1}-D\\(CH_2)_{n_2}-H\\(CH_2)_{n_3}-H\end{matrix}$$

dans lesquels

| | |
|---|---|
| B et D | représentent de l'hydrogène, le groupe phényle ou un groupe phényle substitué par un ou deux substituants de la série nitro, alkyle en $C_1$ à $C_4$, alkoxy en $C_1$ à $C_4$, phényle ou halogène et |

n, $n_1$, $n_2$ et $n_3$ représentent, indépendamment, un nombre de 0 à 5,

caractérisés en ce que le groupe amino en position 6' porte l'un des groupes protecteurs $-SR'$ ou $-CO-A$.

2. Composé suivant la revendication 1, de formule:

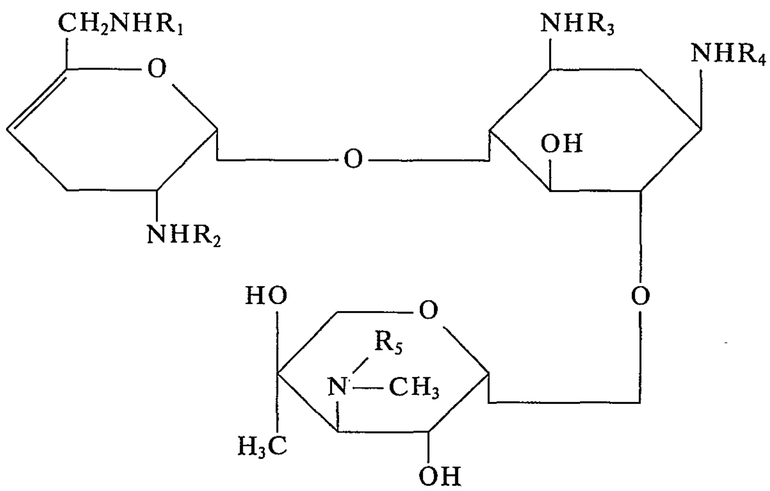

dans laquelle:

| | |
|---|---|
| $R_1$ | est un reste $-SR'$ ou $-CO-A$, et |
| $R_2$, $R_3$, $R_4$ et $R_5$ | désignent l'hydrogène, un reste $-SR'$ ou $-CO-A$, sous réserve qu'un et un seul des restes $R_2-R_5$ représente de l'hydrogène, et |
| R' et A | ont la définition indiquée dans la revendication 1. |

3. La 2',3,3'',6'-tétra-N-(o-nitrophénylsulfényl)-sisomicine.
4. La 1,2',3,6'-tétra-N-acétyl-sisomicine.
5. La 1,3,3'',6'-tétra-N-(tertio-butoxycarbonyl)-sisomicine.
6. La 1,2',3,6'-tétra-N-(éthoxycarbonyl)-sisomicine.
7. La 1,2',3'',6'-tétra-N-(o-nitrophénylsulfényl)-sisomicine.
8. La 1,3,3'',6'-tétra-N-(o-nitrophénylsulfényl)-sisomicine.